# EUROPEAN PATENT APPLICATION

(11) **EP 4 227 402 A1**
(43) Date of publication of application: **16.08.2023**
(21) Application number: 20956526.6
(22) Date of filing: 09.10.2020
(51) Int. Cl.: C12N 5/071, C12Q 1/02

(54) **METHOD FOR INDUCING DIFFERENTIATED CELLS INTO PLURIPOTENT ENDODERM STEM CELLS AND APPLICATION THEREOF**

(71) Applicant: Center for Excellence in Molecular Cell Science, Chinese Academy of Sciences, Shanghai 200031 (CN)
(72) Inventor: CHENG, Xin, Shanghai 200031 (CN); DENG, Xiaogang, Shanghai 200031 (CN); WU, Jiaying, Shanghai 200031 (CN); FU, Tianlong, Shanghai 200031 (CN)
(74) Representative: Mewburn Ellis LLP
(86) International application number: PCT/CN2020/120038
(87) International publication number: WO 2022/073211

(57) **Abstract**

Provided are a method for inducing differentiated cells into pluripotent endoderm stem cells and an application thereof. Specifically, provided is a method for inducing human differentiated cells to differentiate into induced pluripotent endoderm stem cells (smEnSC), comprising the step of: (a) culturing differentiated cells in a culture system under a first culture condition to obtain induced pluripotent endoderm stem cells (smEnSC), the culture system comprising EGF, A83-01, and CHIR99021. Functional induced pluripotent endodermal stem cells have a high differentiation rate and purity.

## Description

### Technical field

The present invention relates to the field of biotechnology and cell therapy. Specifically, the present invention relates to methods for inducing differentiated cells into multipotential endodermal stem cells and applications thereof.

### Background

De-differentiation refers to the loss of specialized functions of differentiated cells in response to external stimuli and their return to earlier developmental stages, and is a common phenomenon in nature. The precursor/stem cells obtained by de-differentiation are important for tissue damage repair, etc.
hEnSCs can expand indefinitely and differentiate into functional endoderm-derived cells in vitro, but their growth is dependent on mouse fibroblasts as trophoblast and high concentration of matrigel, which limits their clinical application.

The de-differentiation of primary parenchymal hepatic cells is particularly evident during their isolation and culture, many functional protein genes, especially the P450 family of monophasic enzymes involved in xenobiotic metabolism, are rapidly lost early in culture. The mechanism of de-differentiation of human primary parenchymal hepatic cells is not yet understood, however, human primary parenchymal hepatic cells are not suitable for the study of de-ifferentiation mechanism because they are not easily obtained, cannot be cultured for a long time in vitro, and are difficult to be genetically manipulated.

Therefore, there is an urgent need to develop a method to induce differentiated cells into multipotent endoderm stem cells (small molecule induced Endoderm Stem Cells, smEnSCs) in this field.

### Summary of the invention

The present invention discloses a method for inducing differentiated cells into multipotent endoderm stem cells (small molecule induced Endoderm Stem Cells, smEnSCs).

The present invention discloses a method for inducing differentiated cells into multipotent endoderm stem cells (small molecule induced Endoderm Stem Cells, smEnSCs) using a combination of small molecule compounds and cytokines.

The present invention also discloses, for the first time in vitro, the use of small molecules to induce de-differentiation of differentiated cells and to establish induced multipotent endoderm stem cell lines (smEnSCs).

The present invention also discloses that, by optimizing the culture method, smEnSCs are able to expand indefinitely in vitro independent of trophoblast cells.

The present invention also discloses that differentiation systems are established for smEnSCs to differentiate in vitro into endoderm-derived cells such as parenchymal hepatic cells, biliary epithelial cell, and intestinal epithelial cell.

In a first aspect of the present invention, it provides a method for inducing differentiation of a human differentiated cell into an induced multipotent endoderm stem cell (smEnSC), comprising the steps of:
(a) culturing a differentiated cell in a culture system under a first culture condition, thereby obtaining an induced multipotent endoderm stem cell (smEnSC); wherein the culture system comprises EGF, A83-01 and CHIR99021.

In another preferred embodiment, the differentiation is de-differentiation.

In another preferred embodiment, the first culture condition comprises a first culture medium.

In another preferred embodiment, the first medium is selected from the group consisting of: SFD, DMEM, DMEM/F12, and a combination thereof.

In another preferred embodiment, the culture system further comprises one or more additives selected from the group consisting of: Ascorbic Acid Phosphate Magnesium, L-glutamine, MTG, bFGF.

In another preferred embodiment, the differentiated cell comprises an adult cell.

In a further preferred embodiment, the adult cell is selected from the group consisting of: a parenchymal hepatic cell, gastric epithelial cell, islet cell, intestinal epithelial cell, and a combination thereof.

In a further preferred embodiment, the parenchymal hepatic cell comprises a primary parenchymal hepatic cell, a parenchymal hepatic cell derived from a stem cell differentiation.

In another preferred embodiment, the adult cell is derived from an adult tissue isolation, in vitro directed differentiation of a stem cell (e.g., endoderm stem cell, induced pluripotent stem cell).

In another preferred embodiment, the endoderm stem cell is derived from a human embryonic stem cell or human induced pluripotent stem cell.

In another preferred example, the pluripotent stem cell is selected from the group consisting of: human embryonic stem cell line (e.g., H9, Hes2), human induced pluripotent stem cell line (e.g., ZRZ-iPSCs, WT6iPSCs), and a combination thereof.

In another preferred example, the endoderm stem cell is selected from the group consisting of: H9, Hes2, ZRZ-iPSC EnSC, WT6iPSC EnSC, and a combination thereof.

In another preferred embodiment, the method further comprises step (b): differentiating the induced multipotent endoderm stem cell (smEnSC) into aan endoderm-derived cell.

In another preferred embodiment, the endoderm-derived cell comprises a parenchymal hepatic cell, biliary epithelial cell, intestinal epithelial cell, islet β cell, and gastric epithelial cell.

In another preferred embodiment, the step (b) does not contain a trophoblast cell.

In a further preferred embodiment, the step (b) comprises:
(b1) culturing an induced multipotent endoderm stem cell in a culture system under a second culture condition, thereby obtaining a parenchymal hepatic cell; or
(b2) culturing an induced multipotent endodermal stem cells in a culture system under a third culture condition, thereby obtaining a bile duct cell; or
(b3) culturing an induced multipotent endodermal stem cells in a culture system under a fourth culture condition, thereby obtaining a small intestinal cell.

In another preferred example, the second culture condition comprises a second culture medium.

In another preferred embodiment, the second culture medium comprises a specialized culture-medium to liver, a maturation medium to liver I, and a maturation medium to liver II.

In another preferred embodiment, the specialized culture-medium to liver is selected from the group consisting of: SFD, DMEM/F12, DMEM, and a combination thereof.

In another preferred example, the maturation medium to liver I is selected from the group consisting of: SFD, DMEM/F12, DMEM, and a combination thereof.

In another preferred embodiment, the maturation medium to liver II is selected from the group consisting of: SFD, HCM, DMEM/F12, DMEM, and a combination thereof.

In another preferred example, the specialized culture-medium to liver further comprises one or more additives selected from the group consisting of:
BMP4, FGF, A83-01, SB431542, IWP2, Dexamethasone, and DMSO.

In another preferred embodiment, the maturation medium to liver I further comprises one or more additions selected from the group consisting of:
HGF, Dexamethasone, OSM, C-E, A83-01, EGFi, VK1.

In another preferred embodiment, the maturation medium to liver II further comprises one or more additions selected from the group consisting of: HGF, Dexamethasone, OSM, C-E, A83-01, EGFi, VK1.

In another preferred example, the third culture condition comprises a third culture medium.

In another preferred example, the third culture medium comprises a bile duct specialized culture-medium and a bile duct maturation medium.

In another preferred embodiment, the third medium is selected from the group consisting of SFD, DMEM/F12, DMEM, William's E, and a combination thereof.

In another preferred example, the third culture condition further contains one or more additions selected from the group consisting of:
FGF10, HGF, TGFβ, EGF, Activin A, and Dexamethasone.

In another preferred example, the fourth culture condition contains a fourth culture medium.

In another preferred embodiment, the fourth culture medium comprises an expansion medium, a posterior intestine specialized culture-medium, a small intestine maturation medium I and a small intestine maturation medium II.

In another preferred embodiment, the fourth medium is selected from the group consisting of: SFD, DMEM/F12, DMEM, and a combination thereof.

In another preferred embodiment, the fourth culture condition further comprises one or more additives selected from the group consisting of:
FGF4, EGF, A83-01, CHIR99321, Wnt3a, R-spondin, EGF, Noggin.

In another preferred example, the expansion medium is selected from the group consisting of: SFD, DMEM/F12, DMEM, and a combination thereof.

In another preferred embodiment, the posterior intestine specialized culture-medium is selected from the group consisting of: SFD, DMEM/F12, DMEM, and a combination thereof.

In another preferred embodiment, the small intestine maturation medium I is selected from the group consisting of: SFD, DMEM/F12, DMEM, and a combination thereof.

In another preferred example, the small intestine maturation medium II is selected from the group consisting of: SFD, DMEM/F12, DMEM, and a combination thereof.

In another preferred embodiment, the expansion medium further comprises one or more additives selected from the group consisting of:
FGF4, EGF, A83-01.

In a further preferred embodiment, the posterior intestine specialized culture-medium further comprises one or more additions selected from the group consisting of:
FGF4, CHIR99321.

In another preferred embodiment, the small intestine maturation medium I further comprises one or more additions selected from the group consisting of:
FGF4, CHIR99321, EGF, Noggin.

In another preferred embodiment, the small intestine maturation medium II further comprises one or more additives selected from the group consisting of: CHIR99321, EGF, Noggin.

In another preferred embodiment, in step (a), the adult cell is cultured under a first culture condition for 3-18 days, preferably, 4-16 days, more preferably, 6-12 days.

In another preferred example, in step (b1), the induced multipotent endoderm stem cell is cultured under a second culture condition for 15-40 days, preferably, 18-36 days, more preferably, 20-25 days.

In another preferred example, in step (b2), the induced multipotent endoderm stem cell is cultured under a third culture condition for 10-30 days, preferably, 15-28 days, more preferably, 15-21 days.

In another preferred embodiment, in step (b3), the induced multipotent endoderm stem cell is cultured under a fourth culture condition for 20-50 days, preferably, 22-45 days, more preferably, 25-40 days.

In another preferred embodiment, the method has one or more features selected from the group consisting of:
(i) a high differentiation rate of the induced multipotent endoderm stem cell, the rate of differentiation being 80-98.5%, preferably, 90-95%;
(ii) during culture, inoculation of 0.5-2×10⁵ adult cells per 1 ml of culture medium yields 0.5-3 ×10⁶ induced multipotent endoderm stem cells.

In another preferred example, the culture system contains no or very little extracellular matrix.

In another preferred embodiment, the extracellular matrix is selected from the group consisting of: Matrigel, Laminin, Basement Membrane Extracts, and a combination thereof

In another preferred example, the content (v/v) of the extracellular matrix in the culture system is 0.5%-5%, preferably, 1%-3%, more preferably, 1%-2%.

In another preferred example, the method comprises a therapeutic and non-therapeutic.

In another preferred example, the density of the adult cell in the culture system is 0.5×10⁶-4×10⁶ cells/plate, preferably, 1×10⁶-2×10⁶ cells/plate.

In another preferred example, the culture system is 10-25 ml, preferably 10-20 ml, most preferably 10-15 ml.

In another preferred example, the ratio M2/M1 of the number M2 of the obtained induced multipotent endoderm stem cell to the number M1 of the adult cell is 5-15, preferably 8-14, more preferably 8-10.

In another preferred example, the induced multipotent endoderm stem cell is a functional induced multipotent endoderm stem cell.

In another preferred embodiment, the endoderm-derived cell is a functional endoderm-derived cell.

In a second aspect of the present invention, it provides an induced multipotent endoderm stem cell, which is prepared and obtained by the method as described in claim 1.

In another preferred embodiment, the induced multipotent endoderm stem cell have one or more characteristics selected from:
(a) 80-90% of the cells are negative for the expression of liver-oriented marker genes;
(b) 85-98% of the cells are positive for gene expression of precursor cell markers;;
(c) 85-98% of cells are positive for endoderm-specific gene expression;
(d) 80-90% of cells are positive for liver precursor cell-specific gene expression;
(e) 85-90% of cells are positive for FOXA1, EpCAM and SOX9 expression;
(f) 85-95% of the cells are positive for CD31 expression.

In another preferred example, the induced multipotent endoderm stem cell has one or more characteristics selected from:
(a) Low or no expression of liver-oriented marker genes;
(b) High expression of a precursor cell marker gene;
(c) High expression of endoderm-specific genes;
(d) High expression of liver precursor cell-specific genes;
(e) High expression of FOXA1, EpCAM and SOX9;
(f) high expression of CD31.

In another preferred embodiment, the liver-oriented marker gene is selected from the group consisting of: *ALB, AAT, CYP3A7, CYP3A4,* and a combination thereof.

In another preferred embodiment, the precursor cell marker gene is selected from the group consisting of: *CDX2, SOX17,* and a combination thereof.

In another preferred embodiment, the endoderm-specific gene is selected from the group consisting of: SOX17, CDX2, FOXA2, and a combination thereof.

In another preferred embodiment, the liver precursor cell-specific gene is selected from the group consisting of: SOX9, TBX3, and a combination thereof.

In another preferred example, the induced multipotent endoderm stem cell is obtained by induction of an adult cell.

In a third aspect of the present invention, it provides an endoderm-derived cell, which is a three-dimensional spherical structure, wherein the size of the sacculus diameter of the endoderm-derived cells is 100-500 µm.

In another preferred embodiment, the endoderm-derived cell has one or more characteristics selected from the following:
(a) parenchymal hepatic cell spheres that are hollow and have a diameter of 150-500 µm;
(b) the bile duct cell structure is a cellular sphere structure or a tubular structure with a cellular sphere diameter of 150-500 µm and a tubular structure with an inner diameter of 20-80 µm and a length of 500 µm-3 mm;
(c) the small intestinal cell structure includes a solid cell sphere, a cell sphere with a lumen, and a tubular structure, wherein the solid cell sphere and the cell sphere with a lumen have a diameter of 100-500 µm and the tubular structure has an inner diameter of 20-80 µm and a length of 500 µm-5 mm.

In another preferred example, the endoderm-derived cell includes a parenchymal hepatic cell, biliary epithelial cell, intestinal epithelial cell, islet β cell, and gastric epithelial cell.

In a fourth aspect of the present invention, it provides a use of the induced multipotent endoderm stem cell as described in a second aspect of the present invention, or the endoderm-derived cell as described in a third aspect of the present invention for the preparation of a drug or formulation, which is used for (a) the treatment of a liver, bile duct-related disease; and/or (b) an intestinal disease; and/or (c) diabetes.

In another preferred example, the liver, bile duct related disease is selected from the group consisting of: inherited metabolic liver disease, slow/acute liver failure, Alagille syndrome, Crigler-Najjar syndrome type 1, and a combination thereof.

In another preferred example, the inherited liver disease is selected from the group consisting of: hepatolenticular degeneration (Wilson's disease, WD), glycogen accumulation disease type Ia, α-antitrypsin deficiency, hematochromatosis, congenital biliary atresia, Citrin protein deficiency, Familial Hypercholesterolemia and a combination thereof.

In another preferred example, the intestinal disease is selected from the group consisting of: intestinal injury, short bowel syndrome, and a combination thereof.

In another preferred example, the diabetes is selected from the group consisting of: type I diabetes, type II diabetes, and a combination thereof.

In a fifth aspect of the present invention, it provides an induction medium, the induction medium comprising a basal medium and an additive; wherein the basal medium is selected from the group consisting of: SFD, DMEM, DMEM/F 12, BDM, and a combination thereof; and wherein the additive comprises EGF, A83-01, and CHIR99021.

In another preferred embodiment, the additive further comprises bFGF, VEGF, EGF, HGF, OSM, and Dexamethasone.

In another preferred embodiment, the additive is selected from one or more additives of the following group: Ascorbic Acid Phosphate Magnesium, L-glutamine, MTG, and bFGF.

In a sixth aspect of the present invention, it provides a use of the induction medium described in the fifth aspect of the present invention for inducing differentiation of a differentiated cell into an induced multipotent endoderm stem cell (smEnSC).

In a seventh aspect of the present invention, it provides a method for screening or identifying a potential therapeutic agent that promote differentiation of a differentiated cell into an induced multipotent endoderm stem cell (smEnSC), comprising the steps of:
(a) in a test group, culturing a differentiated cell in a culture system and in the presence of a test compound for a period of time T1, detecting the number M1 of the induced multipotent endoderm stem cell in the culture system and in the test group;
   and in a control group in which the test compound is not present and other conditions are the same, detecting the number M2 of the induced multipotent endoderm stem cell in the culture system and in the control group; and
(b) comparing M1, M2 as detected in the previous step, thereby determining whether the test compound is a potential therapeutic agent for promoting differentiation of a differentiated cell into an induced multipotent endoderm stem cell;
wherein if M1 is significantly higher than M2, indicating that the test compound is a potential therapeutic agent for promoting differentiation of a differentiated cell into an induced multipotent endoderm stem cell.

In another preferred embodiment, the "significantly higher" means M1/M2 ≥ 2, preferably ≥ 3, more preferably ≥ 4.

In another preferred embodiment, the method is non-diagnostic and non-therapeutic.

It should be understood that within the scope of the present invention, the above-mentioned technical features of the present invention and the technical features described in detail below (such as embodiments) can be combined with each other to form a new or preferred technical solution. Due to space constraints, I will not repeat them here.

### Description of Drawings

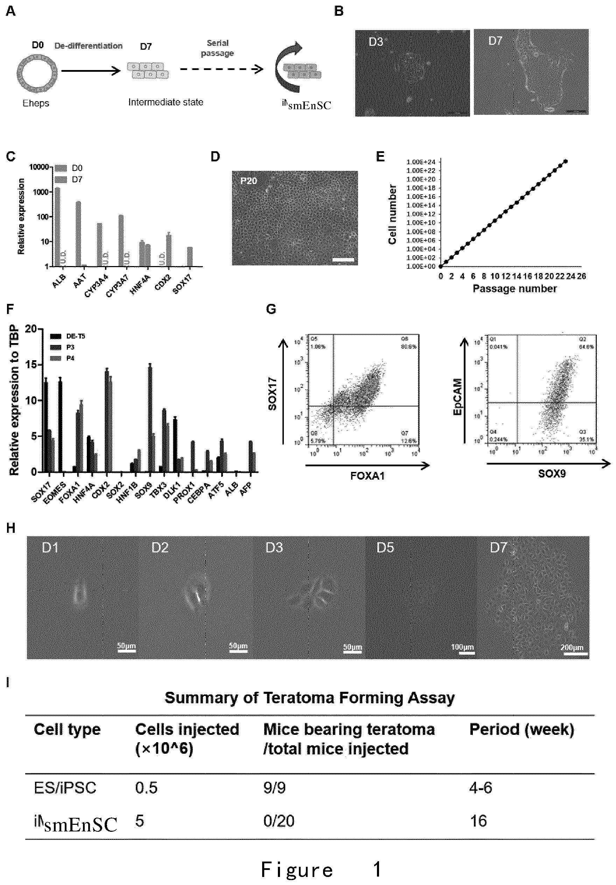

Figure 1 represents the in vitro induction of differentiated parenchymal hepatic cells to de-differentiate and establish smEnSC cell lines; wherein
   (A) Flow diagram of the induction of mature differentiation of Eheps into smEnSCs.
   (B) Diagram of cell morphology during Eheps de-differentiation. D3, D7 indicate the time of induction of de-differentiation, respectively. Scale bar: 200 µm.
   (C) Gene expression changes after 7 days of Eheps de-differentiation. Liver-oriented genes (ALB, AAT, CYP3A7 and CYP3A4) are turned off and precursor cell marker genes (CDX2 and SOX17) are turned on.
   (D) Morphological map of smEnSCs. A homogeneous epithelial-like morphology is shown. Scale bar: 200 µm.
   (E) smEnSCs are able to passage indefinitely in vitro.
   (F) Gene expression of smEnSCs. P3, P4 represent generations of smEnSCs, DE-T5 indicate definitive endoderm. smEnSCs express endoderm-specific genes SOX17, CDX2 and FOXA2, and liver precursor cell-specific genes SOX9 and TBX3.
   (G) Flow cytometric analysis shows that smEnSCs express SOX17 and FOXA1, EpCAM and SOX9.
   (H) smEnSC can be established from single cells and therefore has stemness at the clonal level.
   (I) smEnSCs do not become teratomas in vivo.

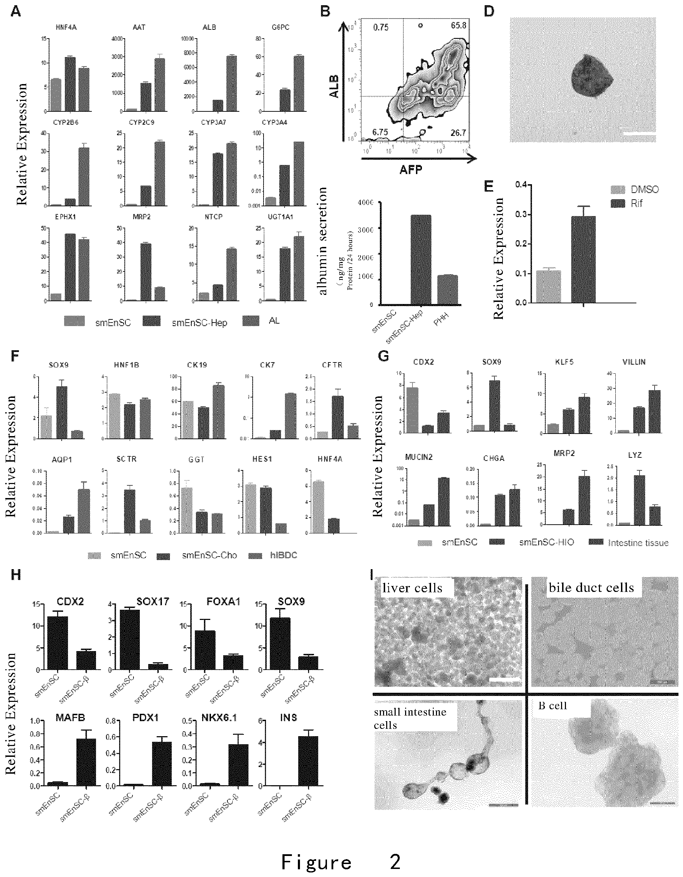

Figure 2 represents in vitro induction of smEnSCs differentiation into endoderm-derived cells; wherein
   (A) smEnSC-Hep gene expression profile. The differentiated and obtained smEnSC-Hep liver-oriented genes such as ALB and CYPs are significantly up-regulated. AL represents adult liver tissue.
   (B) Co-staining of AFP and ALB reveals the efficiency of smEnSCs differentiation into parenchymal hepatic cells in vitro. the AFP positivity rate is up to more than 90% and ALB positivity rate also reaches nearly 70%.
   (C) ELISA detection of smEnSC-Heps capable of secreting albumin.
   (D) PAS staining shows that smEnSC-Heps has the ability of glycogen accumulation. Scale bar: 500 µm.
   (E) smEnSC-Heps are able to respond to rifampicin-induced CYP3A4 expression.
   (F) smEnSCs are induced into bile duct epithelial cells (smEnSC-Cho) in vitro, and qPCR experiments show that they express bile duct epithelial cell marker genes.
   (G) smEnSCs are induced into small intestinal epithelial cells (smEnSC-HIO) in vitro.
   (H) smEnSCs are induced into islet β cells (smEnSC-β cells) in vitro.
   (I) Morphology of in vitro differentiated parenchymal hepatic cells, biliary epithelial cell, small intestinal epithelial cells and islet β-cells from smEnSCs.

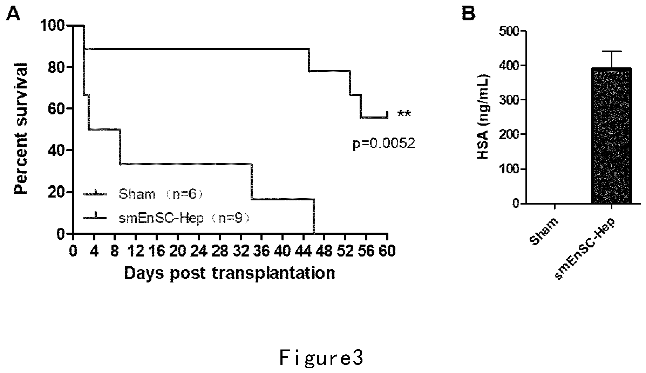

Figure 3 indicates that smEnSC-Hep is able to rescue FRG mice, a model of liver failure; wherein
   (A) smEnSC-Hep transplantation significantly improves the survival of FRG mice. Sham indicates the untransplanted smEnSC-Hep group and smEnSC-Hep indicates the transplanted smEnSC-Hep group.
   (B) Human-derived albumin can be detected in the serum of FRG mice surviving transplantation of smEnSC-Hep.

### DETAILED DESCRIPTION

After extensive and thorough research, the present inventors have unexpectedly discovered for the first time that adult cells cultured in the culture system under the first culture conditions of the present invention can obtain induced multipotent endoderm stem cells (smEnSC), and that the induced multipotent endoderm stem cells of the present invention have a differentiation rate of >90% and induced multipotent endoderm stem cells have a purity of >90%. Moreover, the induced multipotent endoderm stem cells of the present invention can be further differentiated into endoderm-derived cells (such as parenchymal hepatic cells, bile duct cells, small intestine cells, islet β cells, etc.), and in addition, the induced multipotent endoderm stem cells of the present invention can be used for the treatment of liver and bile duct-related diseases and diabetes. On this basis, the present inventors have completed the present invention.

Specifically, the present inventors have invented a method for inducing a differentiated cell into a multipotent endoderm stem cell (small molecule induced Endoderm Stem Cells, smEnSCs) using a combination of small molecule compounds and cytokines. Induced de-differentiation of differentiated cells such as stem cell-derived parenchymal hepatic cells by culturing them with a combination of specific small molecule compounds and cytokines and establish a new endoderm stem cell line with unlimited proliferation independent of trophoblast cells by passaging, which are then further induced to differentiate into parenchymal hepatic cells, biliary epithelial cell, intestinal epithelial cell and islet β cells in vitro.

### Terms

As used herein, the term "A83-01" has the chemical formula C₂₅H₁₉N₅S and the CAS number 909910-43-6;
"C-E" has the chemical formula C₂₇H₂₄F₂N₄O₃ and the CAS number 209986-17-4;
"EGFi" has the chemical formula C₂₂H₂₃N₃O₄.HCl and the CAS number 183319-69-9;
SB431542 has the chemical formula C₂₂H₁₆N₄O₃ and the CAS number 301836-41-9.

As used herein, the term "plate" refers to all sizes of plates, including 58 mm² plates.

As used herein, the term "induced multipotent endoderm stem cells", "smEnSC" refers to induced multipotent endoderm stem cells obtained by the method of the present invention, the induced multipotent endoderm stem cells having one or more of the following characteristics:
(a) Low or no expression of liver-oriented genes;
(b) High expression of precursor cell marker genes;
(c) High expression of endoderm-specific genes;
(d) High expression of liver precursor cell-specific genes;
(e) High expression of FOXA1, EpCAM and SOX9;
(f) High expression of CD31;

### Differentiated cells

In the present invention, differentiated cell refers to a cell type that is further differentiated from a fertilized egg in terms of developmental biology and are of a particular lineage and are unable to proliferate, including mature cells obtained by directed differentiation of stem cells and primary isolated adult cells in the present invention.

In the present invention, differentiated cells include adult cells.

Adult cells refer to primary cells obtained by direct isolation from adult tissues. Adult cells are terminal differentiated cell types and do not have the ability to proliferate.

### Induction culture method for induced multipotent endoderm stem cells

The starting cells of the induced multipotent endoderm stem cells of the present invention are human differentiated cells (including adult cells), which are cultured under a first culture condition, thereby obtaining induced multipotent endoderm stem cells (smEnSC), wherein the culture system under a first culture condition comprises EGF, A83-01 and CHIR99021.

Next, under conditions suitable for culture (e.g., in a culture system containing a second culture medium including the specialized culture-medium to live, the maturation medium to liver I, and the maturation medium to liver II) and additives (the specialized culture-medium to live including BMP4, FGF, A83-01, IWP2, Dexamethasone, and the maturation medium to liver I including HGF, Dexamethasone, OSM, C-E, A83-01, EGFi, the maturation medium to liver II including Dexamethasone, C-E, A83-01, EGFi) or under conditions containing a third culture medium (e.g. SFD) and additives (including FGF10, HGF, TGFβ, dexamethasone) or under conditions containing a fourth culture medium (including an expansion medium, a posterior intestine specialized culture-medium, a small intestine maturation medium I and a small intestine maturation medium II) and additives (expansion medium including FGF4, EGF, A83-01, posterior intestine specialized culture-medium including FGF4, CHIR99321, small intestine maturation medium I including FGF4, CHIR99321, EGF, Noggin, small intestine maturation medium II including CHIR99321, EGF, Noggin), the induced multipotent endoderm stem cells were further cultured to obtain endoderm-derived cells (e.g., parenchymal hepatic cells, bile duct cells, small intestine cells, islet β cells, etc.).

In the present invention, the culture system of the present invention contains no or very little matrigel.

In a preferred embodiment, the SFD (Serum free differentiation medium) comprises the following components:
75% Iscove's modified Dulbecco's medium (IMDM) (Cellgro) supplemented with 0.5x N2 and B27 supplement (Gibco-BRL), 1% penicillin/streptomycin, and 25% Ham's F12 medium (Cellgro) with 0.05% bovine serum albumin.

HCM medium is commercially available (Commercial medium: Lonza: CC-4182) (Ogawa, S., Surapisitchat, J., Virtanen, C., Ogawa, M., Niapour, M., Sugamori, K. S.,... & Zhao, B. (2013). Three-dimensional culture and cAMP signaling promote the maturation of human pluripotent stem cell-derived hepatocytes. development, 140(15), 3285 -3296.).

### Methods for screening or identifying potential therapeutic agents that promote differentiation of differentiated cells into induced multipotent endoderm stem cells (smEnSC)

In the present invention, a method for screening or identifying potential therapeutic agents that promote differentiation of differentiated cells into induced multipotent endoderm stem cells (smEnSC) is provided, comprising the steps of:
(a) in a test group, culturing a differentiated cell in a culture system and in the presence of a test compound for a period of time T1, detecting the number M1 of the induced multipotent endoderm stem cell in the culture system and in the test group;
   and in a control group in which the test compound is not present and other conditions are the same, detecting the number M2 of the induced multipotent endoderm stem cell in the culture system and in the control group; and
(b) comparing M1, M2 as detected in the previous step, thereby determining whether the test compound is a potential therapeutic agent for promoting differentiation of a differentiated cell into an induced multipotent endoderm stem cell;
wherein if M1 is significantly higher than M2, indicating that the test compound is a potential therapeutic agent for promoting differentiation of a differentiated cell into an induced multipotent endoderm stem cell.

In another preferred embodiment, the "significantly higher" means M1/M2 ≥ 2, preferably ≥ 3, more preferably, ≥ 4.

In another preferred embodiment, the method is non-diagnostic and non-therapeutic.

### The main advantages of the present invention include:

(1) For the first time, the present invention has been found to obtain functional induced multipoten endoderm stem cells with very high differentiation rates (>90%) by culturing differentiated cells (e.g., adult cells) in a culture system, and the purity of induced multipotent endoderm stem cells of the present invention is also very high, up to 99%.
(2) The present invention induces de-differentiation of differentiated cells in vitro for the first time using small molecules and establishes induced multipotent endoderm stem cell lines (smEnSCs).
(3) The smEnSCs of the present invention can expand indefinitely in vitro independent of trophoblast cells and can re-differentiate into endoderm-derived cells such as parenchymal hepatic cells, biliary epithelial cell, and intestinal epithelial cell under specific induced differentiation conditions. Therefore, the smEnSC of the present invention is expected to replace hEnSCs in many fields such as regenerative medicine. In addition, the de-differentiation system can also serve as an ideal model for the study of de-differentiation mechanisms.
(4) The stem cell-derived parenchymal hepatic cells of the present invention are easy to obtain, can be cultured in vitro for a long time, and can be genetically manipulated, and thus have the potential to replace human primary parenchymal hepatic cells for the study of the mechanism of de-differentiation.
(5) The present invention uses a combination of small molecule compounds to induce Eheps de-differentiation to establish smEnSCs with low cost and high stability.
(6) The culture of smEnSCs of the present invention is not dependent on trophoblast cells such as MEF, which meets the requirements of clinical treatment.
(7). The smEnSCs of the present invention are able to expand indefinitely in vitro, become non-tumorigenic in vivo, and re-differentiate into endoderm-derived cell types such as parenchymal hepatic cells, biliary epithelial cell, and intestinal epithelial cell under specific in vitro induction conditions. Therefore, they can be used to prepare functional cells that meet clinical needs on an in vitro scale.

The present invention is further described below in connection with specific embodiments. It should be understood that these embodiments are intended to illustrate the invention only and are not intended to limit the scope of the invention. Experimental methods for which specific conditions are not indicated in the following embodiments generally follow conventional conditions, such as those described in Sambrook et al, Molecular Cloning: A Laboratory Manual (New York: Cold Spring Harbor Laboratory Press, 1989), or as recommended by the manufacturer. Percentages and parts are percentages by weight and parts by weight unless otherwise stated.

Reagents and materials used in embodiments of the present invention are commercially available products unless otherwise noted.

### General materials and methods

### Cell culture medium

Induced multipotent endoderm stem cell (smEnSC) medium: SFD medium was supplemented with L-Ascorbic Acid Phosphate Magnesium (50 µg/ml, Wako, cat# 013-19641), L-glutamine (1X, Cellgro), MTG (4.5×10⁻⁴M), bFGF (10 ng/ml), EGF (10 ng/ml), A83-01 (0.5 µM); CHIR99021 (3 µM);
smEnSC parenchymal hepatic cells differentiation medium: the specialized culture-medium to live, SFD medium supplemented with L-Ascorbic Acid Phosphate Magnesium (50 µg/ml, Wako, cat# 013-19641), L-glutamine (1X, Cellgro), MTG (4.5 ×10⁻⁴M), hBMP4 (50 ng/ml); bFGF (20 ng/ml), A83-01 (0.5 µM), IWP2 (5 µM), Dexamethasone (40 ng/ml), DMSO (1% v/v); the maturation medium to liver I, SFD medium supplemented with L-Ascorbic Acid Phosphate Magnesium (50µg/ml, Wako, cat# 013-19641), L-glutamine (1X, Cellgro), MTG (4.5 ×10⁻⁴M), HGF (25ng/ml), Dexamethasone (40ng/ml), OSM (20ng /ml), C-E (0.1uM), A83-01 (0.5uM), EGFi (2uM); the maturation medium to liver II, HCM medium was supplemented with L-Ascorbic Acid Phosphate Magnesium (50µg/ml, Wako, cat# 013-19641), L-glutamine (1X, Cellgro), MTG (4.5×10⁻⁴M), HGF (25ng/ml), Dexamethasone (40ng/ml), C-E (0.1uM), A83-01 (0.5uM), EGFi (2uM).
smEnSC bile duct cell differentiation medium: a bile duct specialized culture-medium, SFD medium supplemented with L-Ascorbic Acid Phosphate Magnesium (50 µg/ml, Wako, cat# 013-19641), L-glutamine (1X, Cellgro), MTG (4.5×10⁻⁴M), ActivinA (50ng/ml). FGF10 (50ng/ml); bile duct maturation medium, SFD medium supplemented with L-Ascorbic Acid Phosphate Magnesium (50µg/ml, Wako, cat# 013-19641), L-glutamine (1X, Cellgro), MTG (4.5×10⁻⁴M), HGF (25ng/ml), TGFβ (5ng/ml), dexamethasone (40ng/ml).
smEnSC small intestine cell differentiation medium: expansion medium, SFD medium supplemented with L-Ascorbic Acid Phosphate Magnesium (50 µg/ml, Wako, cat# 013-19641), L-glutamine (1X, Cellgro), MTG (4.5×10⁻⁴M), FGF4 (10 ng/ml), EGF (10 ng/ml), A83-01 (0.5 µM); posterior intestine specialized culture-medium, SFD medium supplemented with L-Ascorbic Acid Phosphate Magnesium (50 µg/ml, Wako, cat# 013-19641), L glutamine (1X, Cellgro), MTG (4.5 ×10⁻⁴M), FGF4 (500 ng/ml), CHIR99321 (3µM); small intestine maturation medium I, SFD medium supplemented with L-Ascorbic Acid Phosphate Magnesium (50µg/ml, Wako, cat# 013-19641), L-glutamine (1X, Cellgro), MTG (4.5×10⁻⁴M), FGF4 (100 ng/ml), CHIR99321 (3µM), EGF (100 ng/ml), Noggin (100 ng/ml); small intestine maturation medium II, SFD medium supplemented with L-Ascorbic Acid Phosphate Magnesium (50 µg/ml, Wako, cat# 013-19641), L-glutamine (1X, Cellgro), MTG (4.5 ×10⁻⁴M), CHIR99321 (3 µM), EGF (100 ng /ml), Noggin (100 ng/ml).

### Example 1 Induction of Eheps into smEnSC

Differentiated mature Eheps were digested with collagenase B (1 mg/ml, Roche) for 2 hours at 37°C and then digested with 0.25% Tyrisin for 5 minutes, followed by mechanical blowing to single cells, grown in matrigel-coated six-well plates with 1-2×10⁵ cells per well, and cultured with smEnSC medium (Figure 1A). Cell proliferation was clearly observed under light microscope (Figure 1B). qPCR results show that liver-oriented marker genes AAT, ALB, CYP3A7, CYP3A4 are significantly downregulated after 7 days of culture of Eheps in the culture medium of the present invention (Figure 1C). The expression of early endoderm-related genes SOX17 and CDX2 is significantly up-regulated, both of which indicates that substantial degeneration or even loss of the original parenchymal hepatic cells function occurs. Passages are performed every 6 days for the first 2 generations and 3-4 days for the later generations. The number of cells in the starting Eheps is 0.5-2×105, and 1-2×10⁶ cells of smEnSC can be obtained after culture.

The stable smEnSC cell line shows a homogeneous epithelial-like cell morphology (Figure 1D) and in addition is able to expand indefinitely in vitro (Figure 1E). Gene expression analysis shows that smEnSCs express the hepatic enrichment transcription factor HNF4A, as well as the Definitive Endoderm (DE) and hEnSC key transcription factor ription factor EOMES, while there is a significant upregulation of key transcription factors for early liver development such as FOXA1, TBX3, SOX9, CEBPA, and PROX1 (Figure 1F). In addition, smEnSC express CDX2, a key transcription factor for posterior intestinefate determination, but CDX2 is not expressed in DE (Figure 1F). Flow cytometry results show that smEnSC co-expresses SOX17 and FOXA1, EpCAM and SOX9 (Figure 1G). These results suggest that differentiated mature parenchymal hepatic cells undergo de-differentiation in "F" medium, resulting in the establishment of a novel endoderm stem cell line, smEnSC, which is later in development than DE or hEnSCs.

In addition, monoclonal growth assays shows that a single smEnSC is able to grow into a smEnSC clone and establish a lineage (Figure 1H), which validates the stemness of smEnSCs at the clonal level.

The smEnSCs were transplanted into SCID-Beige mice (20 mice, 5×10⁶ cells/mouse) and no in vivo tumorigenesis is detected after 4 months of follow-up (Figure 1I), suggesting that smEnSCs are non-tumor causing in vivo and are a safe seed cell for future cell therapy applications.

### Example 2 Induction of smEnSCs into parenchymal hepatic cells

1×10⁵ smEnSC were first cultured with smEnSC medium for two days, and then replaced with the specialized culture-medium to liver for 4 days. The cells were digested with 0.05% Tyrisin into pellets of 20-50 cells, and the cells of 2 wells were combined into 1 well, 80 µl matrigel was added, and transferred into low adsorption culture plates and continued to incubate for 2 days in the specialized culture-medium to liver. After that, the cells were cultured in the maturation medium to liver I for 12 days and the culture-medium to liver II for 6 days.

The parenchymal hepatic cells (smEnSC-Hep) obtained by differentiation of smEnSCs express a series of proteins specific to adult parenchymal hepatic cells, including the secreted proteins Albumin and AAT, the key enzyme G6PC involved in glycogen accumulation, and the surface marker ASGPR1 in some adult parenchymal hepatic cells. While in terms of transcription factors, smEnSC-Hep expresses a series of liver transcription factors including HNF4A, CEBPA, and HNF6, and the expression levels are similar to those of the adult liver (Figure 2A). Among the P450 family of enzymes that are important markers of maturation of parenchymal hepatic cells, smEnSC-Hep expresses CYP3A4, CYP3A7, CYP2C9, CYP2C19, and CYP2B6 etc. (Figure 2A). In addition, smEnSC-Hep expresses diphasic enzymes such as EPHX1 and a series of transporter proteins, such as NTCP, UGT1A1, MRP2, BCRP, and BSEP etc. (Figure 2A). Flow cytometric analysis reveals that more than 90% of smEnSC-Hep cells are AFP+ and 70% of which are ALB+ (Figure 2B). smEnSC-Heps form cell clusters, "Liver balls" (Figure 2I), which have a specific polar structure, are able to secrete albumin (Figure 2C) and accumulate glycogen (Figure 2D), and significantly upregulate CYP3A4 expression after rifampicin induction (Figure 2E). These results suggest that smEnSC can differentiate efficiently into functional parenchymal hepatic cells in the existing 3D system. 1×10⁵ of smEnSC starting cell mass can yield 2-3×10⁶ parenchymal hepatic cells after a complete differentiation process.

The parenchymal hepatic cells are spherical in structure, hollow spheres with a diameter of about 300 µm.

### Example 3 Induction of smEnSCs into biliary epithelial cell

1×10⁵ smEnSCs were first cultured with smEnSC medium for two days, and then replaced with specialized culture-medium to liver for 4 days. The cells were digested with 0.05% Tyrisin into pellets of 20-50 cells, and the cells of 2 wells were combined into 1 well, adding 80 µl matrigel and transferred to low adsorption culture plates with specialized culture-medium to liver to continue the culture for 2 days. Subsequently, the bile duct specialized culture-medium was replaced and cultured for 4 days. Afterwards, they were transferred to bile duct maturation medium and continued to be cultured for 7 to 10 days.

The biliary epithelial cells smEnSC-Cho obtained by smEnSC differentiation show a three-dimensional organoid morphology (Figure 2I) and are able to form a specific structure with its own lumen after re-adhering on matrigel. And from the results of gene expression analysis, smEnSC-Cho expresses HNF1B and SOX9, key transcription factors of biliary epithelial cells, while the liver transcription factor HNF4A is significantly downregulated (Figure 2F). smEnSC-Cho, expresses a variety of receptor proteins and transporters related to bile duct function, including CTFR, AQP1, SCR, GGT, etc. (Figure 2F). Notch signaling pathway is important for bile duct development, and we have found that Notch signaling is maintained activated in smEnSC and smEnSC-Cho with high expression of its downstream target gene HES1 (Figure 2F). Yes, smEnSC-Cho expresses the important keratins CK19 and CK7 in biliary epithelial cells. 1×10⁵ smEnSCs can obtain 2-3×10⁶ biliary epithelial cells after a complete differentiation process. These results suggest that smEnSCs can differentiate efficiently into biliary epithelial cells in vitro.

The bile duct cell structure is either a cell sphere structure or a tubular structure with a cell sphere diameter of 280 µm and a tubular structure with an inner diameter of 50 µm and a length of 1 mm;

### Example 4 Induction of smEnSCs into small intestinal cells

1×10⁵ smEnSC were first cultured in expansion medium for 2-3 days. Later, they were replaced with posterior intestine specialized culture-medium and cultured for 4 days. After digestion with TrypLE or 0.05% Tyrisin into pellets of 20-50 cells, the cells of 2 wells were combined into 1 well, 80 µ 1 matrigel was added and transferred to low adsorption culture plate with small intestine maturation medium I and continued to be cultured for 3 days. After that, the culture was continued in small intestine maturation medium for 18-24 days.

In terms of gene expression, as with adult small intestinal tissues, HIO obtained by in vitro differentiation of smEnSCs expresses the transcription factors CDX2 and SOX9 for small intestinal fate determination and maintenance (Figure 2G). In addition, HIO obtained by in vitro differentiation of smEnSCs also expresses the Enterocyte-specific marker VILLIN, the enteroendocrine cell-specific marker Chromogranin A (CHGA), and the Paneth cell-specific marker Lysozyme (LYZ); however, Mucin2 (MUC2), a marker of Goblet cell (Figure 2G). Furthermore, HIO formed by three-dimensional suspension differentiation has a highly ordered tissue structure containing internal organ cavity, similar to the organoid structure previously reported to be established from primary small intestinal cells (Figure 2I). Moreover, longer tubular structures are also present in our differentiated system, which has not been reported in studies related to small intestinal differentiation. HIO expresses transporter proteins such as MRP2, which facilitates in vitro mimicry of small intestinal substance absorption, etc. (Figure 2G). These results suggest that smEnSC is able to differentiate into small intestinal tissues with certain structures and multiple small intestinal cell types in vitro.

A starting cell mass of 1×10⁵ smEnSC can obtain 2-3×10⁶ small intestinal cells after a complete differentiation process.

Small intestinal cell structures include solid cell spheres, cell spheres with internal lumen, and tubular structures, wherein the diameter of solid cell spheres and cell spheres with internal lumen is 300 µm, and the internal diameter of tubular structures is 60 µm and 2 mm long.

### Example 5 Induction of smEnSCs into islet β-cells

1×10⁵ smEnSCs were first cultured in expansion medium for 2-3 days and subsequently induced into Pancreatic Foregut, Pancreatic Endoderm, and Pancreatic Progenitor, respectively, under the corresponding culture conditions. after digestion with Dispase or 0.05% Tyrisin into pellets of 20-50 cells, the cells of 2 wells were combined into 1 well, 80 µl matrigel was added, and transferred to low adsorption culture plates for further differentiation into mature islet β cells smEnSC-βCell. qPCR experiments show that smEnSC-βCell expresses islet β cell markers INS, MAFB, PDX1 and NKX6.1, while the smEnSC-specific genes CDX2, SOX17, FOXA1 and SOX9 are significantly downregulated (Figure 2H).

The islet β-cells were three-dimensional solid spherical structures with structural features such as 200 µm-500 µm in diameter (Figure 2I).

### Example 6 smEnSC-Hep transplanted FRG mice experiment

The withdrawal of NTBC from drinking water was started 6-7 days before FRG mice were transplanted. smEnSC-heps were digested by collagenase B (1 mg/ml) for 1 h and then treated with 0.25% Trypsin-EDTA for 5 min. The digested cells were filtered through a 70 µm filter membrane and finally 1×10⁶ Eheps were resuspended with 100-150 µl PBS, placed on ice and cells were transplanted from the spleen. Mice were weighed every 3 days after transplantation and death was recorded. Mice that were not transplanted with cells and lost 30% of their body weight were served as negative controls. Mice that were still alive 8 weeks after cell transplantation were processed and blood and liver samples were collected for later analysis.

The results show that human-derived albumin is detectable in the serum of FRG mice after transplantation with smEnSC-Hep (Figure 3B), and mouse survival is significantly improved (Figure 3A). These results suggest that smEnSC-Hep has a better therapeutic effect on diseases such as liver failure.

### Discussion

Cell de-differentiation, a biological phenomenon prevalent in nature, plays an active role in individual damage repair, etc. The use of terminally differentiated cells to dedifferentiate into proliferative cells is a new idea to establish novel stem cell lines. The molecular mechanism of dedifferentiation is currently unknown due to the lack of ideal in vitro cell models.

In the present invention, the study of dedifferentiation using hEnSCs-derived parenchymal hepatic cells Eheps shows that the inhibition of TGFβ signaling pathway and the activation of MAPK signaling pathway have important roles in the dedifferentiation of differentiated cells.

By manipulating these signaling pathways, the present invention uses differentiated cells to establish novel induced multipotent endoderm stem cell lines (smEnSCs) that can be stably passaged in vitro. This cell line can efficiently differentiate into endoderm-derived cells such as liver parenchyma, bile duct epithelium and small intestine, providing novel seed cells for regenerative medicine. Compared with hEnSCs, the culture of smEnSCs is not dependent on trophoblast cells and meets the requirements of clinical applications.

In summary, the induction dedifferentiation of Eheps by small molecule compounds can establish a novel endodermal stem cell line, smEnSCs, which provides an ideal in vitro model for studying the mechanism of primary liver parenchymal cell dedifferentiation and provides novel seed cells for regenerative medicine.

The present invention utilizes transcriptome analysis at the single-cell level combined with epigenomic analysis to investigate the dynamic molecular regulatory mechanisms during the above-mentioned dedifferentiation process. The use of single-cell sequencing technology to study the dynamics of the Ehep dedifferentiation process enables, on the one hand, to track the changes in each cell group during the process and infer which specific cellular subpopulations are able to respond to exogenous induction signals; on the other hand, it can comprehensively reveal the key signaling pathways in the dedifferentiation process.

Other existing methods for parenchymal hepatic cell culture have multiple defects in terms of cell proliferation and serum dependence, which make it difficult to meet the application requirements. However, the dedifferentiation of parenchymal hepatic cell under this induction condition does not yield endoderm stem cells equivalent to hEnSC or smEnSC stages, suggesting that the epigenetic and transcriptional status of differentiated cells may affect the process and outcome of dedifferentiation.

In summary, the present invention uses hEnSC-derived Eheps to mimic the dedifferentiation process of primary parenchymal hepatic cells in vitro and to establish smEnSCs, a novel endoderm stem cell line with unlimited expansion capacity and multiple differentiation potentials. The present invention is expected to enable the in vitro dedifferentiation and establishment of stably proliferating tissue-specific stem cells from human adult parenchymal hepatic cells as well as other terminally differentiated cells using a similar approach, providing a new source of cells for regenerative medicine applications.

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. A method for inducing differentiation of a human differentiated cell into an induced multipotent endoderm stem cell (smEnSC), comprising the steps of:
(a) culturing a differentiated cell in a culture system under a first culture condition, thereby obtaining an induced multipotent endoderm stem cell (smEnSC); wherein the culture system comprises EGF, A83-01 and CHIR99021.

2. The method of claim 1, wherein the differentiation is de-differentiation.

3. The method of claim 1, wherein the differentiated cell comprises an adult cell.

4. The method of claim 3, wherein the adult cell is selected from the group consisting of: a parenchymal hepatic cell, gastric epithelial cell, islet cell, intestinal epithelial cell, and a combination thereof.

5. An induced multipotent endoderm stem cell, which is prepared and obtained by the method as described in claim 1.

6. An endoderm-derived cell, which is a three-dimensional spherical structure, wherein the size of the sacculus diameter of the endoderm-derived cells is 100-500 µm.

7. Use of the induced multipotent endoderm stem cell of claim 5, or the endoderm-derived cell of claim 6 for the preparation of a drug or formulation, which is used for (a) the treatment of a liver, bile duct-related disease; and/or (b) an intestinal disease; and/or (c) diabetes.

8. An induction medium, the induction medium comprising a basal medium and an additive; wherein the basal medium is selected from the group consisting of: SFD, DMEM, DMEM/F12, BDM, and a combination thereof; and wherein the additive comprises EGF, A83-01, and CHIR99021.

9. Use of the induction medium of claim 8 for inducing differentiation of a differentiated cell into an induced multipotent endoderm stem cell (smEnSC).

10. A method for screening or identifying a potential therapeutic agent that promote differentiation of a differentiated cell into an induced multipotent endoderm stem cell (smEnSC), comprising the steps of:
(a) in a test group, culturing a differentiated cell in a culture system and in the presence of a test compound for a period of time T1, detecting the number M1 of the induced multipotent endoderm stem cell in the culture system and in the test group;
and in a control group in which the test compound is not present and other conditions are the same, detecting the number M2 of the induced multipotent endoderm stem cell in the culture system and in the control group; and
(b) comparing M1, M2 as detected in the previous step, thereby determining whether the test compound is a potential therapeutic agent for promoting differentiation of a differentiated cell into an induced multipotent endoderm stem cell;
wherein if M1 is significantly higher than M2, indicating that the test compound is a potential therapeutic agent for promoting differentiation of a differentiated cell into an induced multipotent endoderm stem cell.
